# EUROPÄISCHE PATENTSCHRIFT

(11) **EP 3 705 029 B1**
(45) Veröffentlichungstag und Bekanntmachung des Hinweises auf die Patenterteilung: **29.03.2023**
(21) Anmeldenummer: 20158196.4
(22) Anmeldetag: 19.02.2020
(51) Int. Cl.: A61B 3/103, A61B 3/107, A61B 3/10, A61B 3/00, A61B 3/18

(54) **VERFAHREN UND SEHPRÜFSYSTEM ZUM ÜBERPRÜFEN DER AUGEN**
VISION TESTING SYSTEM AND METHOD FOR TESTING EYES
PROCÉDÉ ET SYSTÈME D'EXAMEN DE LA VUE DESTINÉS AU CONTRÔLE DES YEUX

(30) Priorität: 07.03.2019 DE 102019105756
(43) Veröffentlichungstag der Anmeldung: 09.09.2020
(73) Patentinhaber: Oculus Optikgeräte GmbH, 35582 Wetzlar (DE)
(72) Erfinder: STEINMÜLLER, Andreas, 35435 Wettenberg (DE)
(74) Vertreter: advotec.

(56) Entgegenhaltungen:
- EP-A1- 3 210 526
- EP-A1- 3 222 204
- WO-A1-2015/044364
- DE-A1-102017 210 577
- US-A1- 2017 189 233

## Beschreibung

Die Erfindung betrifft ein Verfahren zum Überprüfen der Augen eines Probanden mit den Merkmalen des Oberbegriffs des Anspruchs 1 und ein Sehprüfsystem mit den Merkmalen des Oberbegriffs des Anspruchs 17.

Refraktive Messvorrichtungen sind hinreichend bekannt und dienen regelmäßig zur Bestimmung eines Refraktionswertes eines Auges eines Probanden. Der Refraktionswert kann zur Beurteilung einer optischen Fehlsichtigkeit des Auges herangezogen werden. Eine häufige Form der Fehlsichtigkeit ist die Kurzsichtigkeit, bei der eine Bildebene vor der Netzhaut liegt, wodurch ein unscharfer Seheindruck entsteht. Bei der Kurzsichtigkeit (Myopie) wird zwischen verschiedenen Formen unterschieden. Bei der sogenannten Achsenmyopie ist eine axiale Länge des Auges erhöht. Bei der Refraktionsmyopie liegt eine erhöhte Brechkraft der refraktiven Teile des Auges, beispielsweise Hornhaut oder Linse, vor. Die erhöhte Brechkraft kann beispielsweise durch eine verstärkte Krümmung einer refraktiven Fläche, wie der Hornhaut, verursacht sein. Auch kann ein Brechungsindex der Linse verändert sein, sodass sich hieraus eine erhöhte Brechkraft ergibt. Kurzsichtigkeit kann sich bereits im Kindesalter durch verschiedene Umgebungsfaktoren, wie Sehaufgaben im Nahbereich des Auges, entwickeln oder mit dem fortschreitenden Alter entstehen, beispielsweise durch Nachlassen der Akkommodationsfähigkeit des Ziliarmuskels mit der Linse. Neben Medikamenteneinnahme können auch Krankheiten, wie Diabetes mellitus oder eine genetische Disposition eine Ursache für Kurzsichtigkeit sein.

Mit einer refraktiven Messvorrichtung kann ein Refraktionswert in Dioptrien (dpt) vergleichsweise einfach objektiv bestimmt werden. So ist aus der DE 102 006 017 389 A1 ein Sehprüfsystem bekannt, welches aus einer refraktiven Messvorrichtung und einer topografischen Messvorrichtung gebildet ist. Bei der refraktiven Messvorrichtung handelt es sich hier um ein Autorefraktometer, welches mit der topografischen Messvorrichtung, die in Art eines Keratometers ausgebildet ist, kombiniert ist. Mit dem Keratometer kann eine Topografie der Hornhaut bestimmt werden, wodurch Informationen über eine mögliche Ursache einer Kurzsichtigkeit, beispielsweise einem eventuell vorhandenen Katarakt, gewonnen werden können.

Dennoch ist die Beurteilung der so erhaltenen Refraktions- und Topographiedaten schwierig, da die Refraktionsmessung fehlerhaft sein kann, und auch andere Einflussfaktoren für bestimmte Refraktionswerte ursächlich sein können. Eine fehlerhafte Refraktionsmessung kann sich beispielsweise dadurch ergeben, dass der Proband während der Messung mit dem Auge im Nahbereich akkommodiert. Um dies auszuschließen kann der Ziliarmuskel medikamentös gelähmt werden (Zykloplegie), was jedoch zeitaufwendig und für den Probanden unangenehm ist.

Bei der Achsenmyopie kommt es zu einer Zunahme der zentralen Achslänge des Auges, was auch zu einer Dehnung der Wandstrukturen des Auges, insbesondere der Netzhaut führt. Diese Dehnung betrifft dann auch die periphere (äquatoriale) Netzhaut. Im Bereich der peripheren Netzhaut kann daher der periphere Refraktionswert von einem zentral auf der Sehachse gemessenen zentralen Refraktionswert abweichen. Erfolgt eine Korrektur einer Achsenmyopie mit einer Brille oder einer Kontaktlinse, so wird auf der Sehachse eine Bildebene bzw. ein Fokus auf die Netzhaut verschoben, insbesondere im Makulabereich. Da jedoch im peripheren Bereich der Netzhaut ein Refraktionswert bei einer Achsenmyopie regelmäßig abweichend ist, wird in dem peripheren Bereich durch die Korrektur mit der Brille oder Kontaktlinse die Bildebene bzw. ein Fokus hinter die Netzhaut verschoben. Da die Bildebene bei einem gesunden Auge an die Form der Netzhaut angepasst ist, ist sie schalenförmig bzw. entspricht einer Bildschale. Bei der Korrektur mit der Brille oder der Kontaktlinse liegt diese Bildschale dann nur im Makulabereich auf der Netzhaut und ist im peripheren Bereich der Netzhaut hinter die Netzhaut verschoben, so dass die Myopie im peripheren Bereich dann überkorrigiert wurde.

Während eines Augenwachstums im Kindesalter kann eine derartige Korrektur einer Achsenmyopie jedoch dazu führen, dass ein Wachstum des Augapfels derart beeinflusst wird, dass eine Länge des Augapfels weiter zunimmt und damit die Achsenmyopie schneller fortschreitet. Da in dieser Wachstumsphase das Auge zum Brennpunkt der Linse hin wächst, wird allgemein angenommen, dass eine Sehhilfe eine Wachstumskorrektur des Auges verhindert. Eine Verlagerung der Bildschale im peripheren Bereich hinter die Netzhaut begünstigt offenbar diese unerwünschte Wachstumsentwicklung des Auges. Es kann davon ausgegangen werden, dass ein Fortschreiten einer Achsenmyopie verlangsamt werden kann, wenn mit einer Sehhilfe die Bildschale im peripheren Bereich der Netzhaut vor die Netzhaut verlagert werden kann.

Aus der EP 3 222 204 A1 ist ein Sehprüfsystem mit verschiedenen Messvorrichtungen innerhalb eines Geräts bekannt. Unter anderem sind eine erste Messvorrichtung zur Messung einer Achslänge eines Auges, eine zweite topographische Messvorrichtung zur Bestimmung einer Topographie der Hornhaut sowie eine dritte refraktive Messvorrichtung vorgesehen. Weiter umfasst das Sehprüfsystem eine Fixationseinrichtung mit einem Projektor, einer Linse zur Fokussierung und einem Linsenpaar zur Korrektur eines Astigmatismus des Probanden. Zunächst ist vorgesehen, eine objektive Refraktionsmessung vorzunehmen und danach das Linsenpaar durch gegenseitiges Verdrehen der Linsen so einzustellen, dass ein Astigmatismus des zu messenden Auges korrigiert ist. Dem Probanden wird nun nachfolgend eine Fixationsmarke oder ein Sehzeichen über den Projektor dargeboten. Die Fixationsmarke wird über Spiegel in einen zentralen Strahlengang des Sehprüfsystems eingekoppelt.

Die US 2017/189233 A1 beschreibt ein Sehprüfsystem mit drei Messvorrichtungen und einer Fixationseinrichtung. Mit dem Sehprüfsystem kann eine Hornhautoberfläche mit einer OCT-Messvorrichtung gemessen werden, wobei eine Fixationsmarke so angeordnet werden kann, dass das gemessene Auge eines Probanden in einem definierten Winkel fixiert ist.

Die DE 10 2017 210 577 A1 offenbart eine Achslängenmessung an einem Auge unter Berücksichtigung von Brechzahlen der Augenmedien. Die Achslängenmessung dient insbesondere zur Berechnung einer zu implantierenden Intraocularlinse.

Der vorliegenden Erfindung liegt daher die Aufgabe zugrunde, ein Verfahren und ein Sehprüfsystem vorzuschlagen, mit dem Refraktionseigenschaften eines Auges genauer bestimmbar sind.

Diese Aufgabe wird durch ein Verfahren mit den Merkmalen des Anspruchs 1 sowie durch ein Sehprüfsystem mit den Merkmalen des Anspruchs 17 gelöst.

Das erfindungsgemäße Verfahren zum Überprüfen der Augen eines Probanden wird mit einem Sehprüfsystem durchgeführt, welches eine erste interferometrische Messvorrichtung, eine zweite topografische Messvorrichtung, eine dritte refraktive Messvorrichtung und eine Verarbeitungseinrichtung umfasst, wobei mit der ersten Messvorrichtung eine zentrale Achslänge und eine periphere Achslänge eines Auges des Probanden gemessen werden, wobei mit der zweiten Messvorrichtung eine Krümmung der Hornhaut des Auges gemessen wird, wobei mit der dritten Messvorrichtung eine Refraktionseigenschaft des Auges gemessen wird, wobei mit der Verarbeitungseinrichtung Messdaten der Messung der ersten, zweiten und dritten Messvorrichtung bearbeitet werden, wobei die Verarbeitungseinrichtung die Messdaten ausgibt, wobei das Sehprüfsystem eine Fixationseinrichtung aufweist, wobei das Auge mittels der Fixationseinrichtung wahlweise zur Messung der zentralen Achslänge oder der peripheren Achslänge relativ zum Sehprüfsystem fixiert wird, wobei eine periphere Fixationsmarke der Fixationseinrichtung dargestellt und von dem Auge fixiert wird, derart, dass eine Sehachse des Auges von einer Messachse der ersten Messvorrichtung abweicht.

Neben der objektiven Bestimmung des Refraktionswerts können bei dem Verfahren Messdaten für Topographie bzw. Krümmung der Hornhaut sowie die Achslänge des Auges ermittelt werden. Aufgrund dieser dann gleichzeitig vorliegenden Messdaten wird es für eine untersuchende Person einfacher möglich, den gemessenen Refraktionswert zu beurteilen. Wesentlich für die Erfindung ist es, dass als Achslänge die zentrale Achslänge, die im Bereich der Sehachse liegt, und die periphere Achslänge, die außerhalb des Bereichs der Sehachse liegt und in dem peripheren Bereich der Netzhaut außerhalb der Fovea centralis gemessen wird. Sofern eine Achsenmyopie vorliegt, ergeben sich dann regelmäßig unterschiedliche Messdaten für die zentrale Achslänge und die periphere Achslänge. Eine untersuchende Person ist dann anhand der von der Verarbeitungseinrichtung ausgegebenen Messdaten in der Lage zu beurteilen, ob eine Achsenmyopie vorliegt bzw. inwieweit die Netzhaut innerhalb der Fovea centralis gegenüber der Netzhaut im peripheren Bereich verformt ist. Durch die Messung der zentralen Achslänge und der peripheren Achslänge kann so eine umfassendere Beschreibung des untersuchten Auges erhalten werden. Mit den gewonnenen Messdaten können nachfolgend verbesserte optische Korrekturen bzw. Sehhilfen berechnet oder ausgewählt werden.

Bei der Messung der zentralen Achslänge kann die Sehachse des Auges mit einer optischen Messachse der ersten Messvorrichtung fluchten, wobei bei der Messung der peripheren Achslänge die Sehachse des Auges relativ zu der optischen Messachse der ersten Messvorrichtung um einen Winkel α > 0° geneigt sein kann. Die Sehachse verläuft regelmäßig ausgehend von der Fovea im zentralen Bereich der Netzhaut. Ein peripherer Bereich der Netzhaut beginnt in einer äußeren Randzone der Makula bzw. Perifovea. Die periphere Achslänge wird daher ausgehend von dem peripheren Bereich der Netzhaut gemessen. Prinzipiell kann die Messung der peripheren Achslänge bereits außerhalb der Fovea centralis mit einem Winkel α von ≥ 1°40', außerhalb der Fovea mit einem Winkel α ≥ 5°, außerhalb der Parafovea mit einem Winkel α ≥ 8°20' oder außerhalb der Perifovea mit einem Winkel α ≥ 18°20' erfolgen.

Die Krümmung der Hornhaut des Auges und die Refraktionseigenschaft des Auges kann bei mit der optischem Messachse der ersten Messvorrichtung fluchtenden Sehachse des Auges und/oder bei relativ zu der optischen Messachse der ersten Messvorrichtung um den Winkel α ≥ 0° geneigter Sehachse des Auges gemessen werden. Wenn das Auge relativ zum Sehprüfsystem zur Messung der zentralen Achslänge und zur Messung der peripheren Achslänge beispielsweise mit dem Winkel α geneigt wird, kann auch die Krümmung der Hornhaut und die Refraktionseigenschaft des Auges nicht nur bei der Messung der zentralen Achslänge sondern auch bei der Messung der peripheren Achslänge gemessen werden. Es ergibt sich insbesondere dann auch ein Messwert für die Refraktionseigenschaft des Auges für den peripheren Bereich der Netzhaut an dem die periphere Achslänge gemessen wird. So wird eine noch genauere Prüfung des optischen Systems des Auges möglich.

Bei der Messung der peripheren Achslänge kann die Sehachse des Auges relativ zu der optischen Messachse der ersten Messvorrichtung um einen Winkel α von 20°, ± 10° geneigt sein. Diese Vergleichsweise starke Neigung der Sehachse relativ zu der optischen Messachse kann der periphere Bereich der Netzhaut bzw. der entsprechend gekrümmten Bildschale des Auges vorteilhaft untersucht werden. Die in diesem Bereich gewonnenen Messdaten ermöglichen eine Bestimmung bzw. Bewertung einer Kurzsichtigkeit oder Achsmyopie durch eine untersuchende Person.

Die periphere Achslänge kann für verschiedene Neigungen der Sehachse des Auges relativ zu der optischen Messachse der ersten Messvorrichtung gemessen werden, wobei der Winkel α in Schritten von 5° verändert werden kann. So ist es dann beispielsweise möglich, die periphere Achslänge des Auges an vielen Punkten der Netzhaut zu messen. Diese Messpunkte können auch auf konzentrischen Kreisen relativ zur Sehachse verteilt liegen, so dass eine große Anzahl von Messdaten der jeweiligen peripheren Achslängen erhalten wird. Wenn bei jeder einzelnen Messung der jeweiligen peripheren Achslänge ergänzend die Refraktionseigenschaft des Auges gemessen wird, wird eine vollumfängliche Erfassung des optischen Systems des Auges über die Messdaten möglich.

Mittels einer Fixationseinrichtung des Sehprüfsystems kann eine für das Auge fokusierbare periphere Fixationsmarke dargestellt werden, wobei das Auge die Fixationsmarke fokussieren kann und eine Fixierung des Auges relativ zum Sehprüfsystem so erfolgen kann. Die periphere Fixationsmarke kann so angeordnet sein, dass eine optische Achse des Auges bzw. die Sehachse des Auges nicht mit der Messachse der ersten Messvorrichtung fluchtet und so von dieser abweicht. Die Fixationsmarke kann beispielsweise eine Leuchtdiode sein, über die dem betreffenden Probanden ein Sehreiz dargeboten wird, der dann eine Ausrichtung des Auges relativ zu der Messachse der ersten Messvorrichtung in der gewünschten Weise bewirkt. Dass die periphere Fixationsmarke sich dann in einem Nahbereich des Auges befindet, und auch eine Akkommodation des Auges auf die periphere Fixationsmarke erfolgt, ist für eine Messung der peripheren Achslänge nicht von Bedeutung. Im Gegensatz dazu wäre die Bestimmung einer Refraktionseigenschaft mit einem auf einen Nahbereich akkommodierten Auge nur fehlerhaft möglich, so dass dann eine Lähmung des Ziliarmuskels (Zykloplegie) erforderlich wäre. Eine Bestimmung des refraktiven Gesamtzustandes des Auges mit Messwerten für die periphere Achslänge ist so auch ohne Zykloplegie möglich.

Mittels einer Fixationseinrichtung des Sehprüfsystems kann eine für das Auge im Unendlichen fokussierbare zentrale Fixationsmarke dargestellt werden, wobei das Auge die Fixationsmarke fokussieren und eine Fixierung des Auges relativ zum Sehprüfsystem erfolgen kann. Wesentlich für eine genaue Messung des Sehprüfsystems ist eine lagegerechte Anordnung des Auges relativ zu einer Messachse des Sehprüfsystems. Vorteilhaft fluchtet dabei eine optische Achse des Auges mit der Messachse des Sehprüfsystems. Die Ausrichtung des Auges kann dabei dadurch erfolgen, dass dem Probanden die Fixationsmarke dargeboten wird, die der Proband im Unendlichen fokussieren kann. Die Fixationsmarke kann beispielsweise eine bildhafte Darstellung eines Gegenstandes sein. Die bilderhafte Darstellung kann durch einen in einen Strahlengang des Sehprüfsystems eingekoppelten Bildschirm erfolgen. Wesentlich ist, dass sich die bildhafte Darstellung für das Auge des Probanden im Unendlichen befindet, sodass der Ziliarmuskel des Auges bei der Messung mit dem Sehprüfsystem vollständig entspannt ist.

Vorteilhaft kann die Überprüfung der Augen dann auch ohne Verabreichung von Zykloplegika durchgeführt werden. Eine Überprüfung der Augen wird dadurch einfacher und schneller möglich.

Mittels einer Fixationseinrichtung des Sehprüfsystems können auch verschiedene Akkommodationszustände des Auges des Probanden erzeugt werden. Dabei kann dann für jeden der Akkommodationszustände eine zentrale Achslänge und/oder eine periphere Achslänge des Auges sowie eine Refraktionseigenschaft des Auges gemessen werden. Die für die verschiedenen Akkommodationszustände bzw. verschiedenen Blickpositionen des Auges gewonnenen Messwerte können von der Verarbeitungseinrichtung zur Erzeugung einer umfassenden optischen Beschreibung des Auges verwendet werden. Mit diesen Messwerten können verbesserte optische Korrekturen, beispielsweise Brille, Kontaktlinse, Intraokularlinse, phakic IOL, Laser-OP, berechnet werden.

An dem Auge kann eine Messung mit der ersten, zweiten und dritten Messvorrichtung zeitgleich durchgeführt werden. Die Messung der Achslänge erfolgt jedoch in zwei Schritten.

Wenn die Achslänge des Auges, die Krümmung der Hornhaut des Auges und die objektive Refraktionseigenschaft beziehungsweise ein Refraktionswert des Auges in beispielsweise Dioptrie gleichzeitig gemessen wird, können Messfehler, wie sie bei aufeinanderfolgenden Messungen mit verschiedenen Messgeräten auftreten können, ausgeschlossen werden. Bei der zeitgleichen Messung mit der ersten, zweiten und dritten Messvorrichtung beziehen sich alle gewonnenen Messdaten auf einen zu diesem Zeitpunkt vorliegenden Zustand des Auges, was eine Vergleichbarkeit der Messdaten bei gleichen Bedingungen ermöglicht. Bei der Verwendung unterschiedlicher Geräte oder bei aufeinanderfolgenden Messungen ist das Auge in Folge von Augenbewegungen stets unterschiedlich zu einer Messachse ausgerichtet oder befindet sich in unterschiedlichsten Akkommodationszuständen.

Mittels der Verarbeitungseinrichtung kann ein Vergleich der zentralen Achslänge mit der peripheren Achslänge durchgeführt und ein Ergebnis des Vergleichs zusammen mit den Messdaten ausgegeben werden. Der Vergleich erfolgt dabei mittels der Verarbeitungseinrichtung, die ein Ergebnis des Vergleichs ausgibt und so einer untersuchenden Person eine einfachere Beurteilung der gewonnenen Messdaten ermöglicht. Die Verarbeitungseinrichtung umfasst dabei Mittel zur Datenverarbeitung und Darstellung, wie beispielsweise einen Computer und einen Bildschirm.

Gleichwohl kann auch mittels der Verarbeitungseinrichtung ein Vergleich der zentralen Messdaten mit der peripheren Achslänge durchgeführt und ein Ergebnis des Vergleichs zusammen mit den Messdaten ausgegeben werden. Die zentralen Messdaten können dann neben der zentralen Achslänge Messdaten zum objektiven Refraktionswert und zur Topographie bzw. Krümmung der Hornhaut umfassen. Aufgrund dieser dann vorliegenden Messdaten wird es für eine untersuchende Person einfacher möglich, den gemessenen Refraktionswert zu beurteilen.

Mittels der Verarbeitungseinrichtung kann durch den Vergleich eine Bestimmung eines Grades der Refraktion erfolgen. Mit dem Grad der Refraktion kann eine Einstufung der ermittelten Messwerte der Achslängen anhand der Abweichung von den übrigen Messdaten erfolgen. Ein Grad einer objektiven Refraktion kann anhand der physikalischen Größen der Messwerte bzw. Messdaten von der Verarbeitungseinrichtung ermittelt werden. Dabei kann eine unterschiedliche Gewichtung der Messwerte vorgenommen werden oder die Messwerte können ins Verhältnis gesetzt werden. Der Grad der objektiven Refraktion kann von der Verarbeitungseinrichtung ausgegeben werden, beispielsweise auf einem Bildschirm, sodass eine noch einfachere Beurteilung der Refraktionseigenschaften des Auges möglich wird.

Mittels der Verarbeitungseinrichtung kann aus den Messdaten eine Brechzahl und/oder ein Brechzahlgradient der Linse des Auges des Probanden bestimmt werden. Eine Brechzahl der Linse des Auges ändert sich mit dem Alter eines Probanden. Auch kann die Brechzahl der Linse des Auges an verschiedenen Stellen der Linse voneinander abweichen. Durch die Messung der peripheren Achslänge und zumindest der Refraktionseigenschaft des Auges im peripheren Bereich wird es möglich die auch Brechzahl der Linse des Auges, vorzugsweise die mittlere bzw. gemittelte Brechzahl, in diesem Bereich zu bestimmen. Der Brechzahlgradient ergibt sich dann mit Bezug auf die zentral ermittelten Messdaten des Auges.

Die Verarbeitungseinrichtung kann eine Datenbank mit Normaldaten aufweisen, wobei mittels der Verarbeitungseinrichtung ein Vergleich der Messdaten mit den Normaldaten durchgeführt und ein Ergebnis des Vergleichs ausgegeben werden kann. Wenn die Verarbeitungseinrichtung den Vergleich der Messdaten mit den Normaldaten durchführt, kann anhand des Ergebnisses des Vergleichs bzw. der jeweiligen Differenz eines Messwertes mit einem Normalwert eine Abweichung leicht beurteilt werden.

Wenn beispielsweise ein Messwert für die Krümmung der Hornhaut von einem Normalwert stark abweicht, kann die untersuchende Person einen ebenfalls vom Normalwert abweichenden Refraktionswert leichter interpretieren. Im vorgenannten Beispiel kann dann die Krümmung der Hornhaut ursächlich für den Refraktionswert sein. Weiter kann eine zu große Achslänge als eine Ursache für einen Refraktionswert von einer untersuchenden Person bestimmt werden. Liegen für die Krümmung der Hornhaut und für die Achslänge des Auges keine von einem Normalwert abweichenden Messwerte vor, kann beispielsweise ein Brechungsindex der Linse verändert sein. Insgesamt wird es durch die zeitgleiche Gewinnung der Messwerte möglich einfach und schnell genaue Messwerte zu erhalten und diese mit Normalwerten zu vergleichen.

Als Normaldaten können Messdaten von Augen einer Normalpopulation mi einer zentralen Achslänge und/oder einer peripheren Achslänge eines Auges, einer Krümmung der Hornhaut des Auges und einer Refraktionseigenschaft des Auges verwendet werden.

Die Normaldaten können dem fünfzigsten Perzentil einer Vergleichsgruppe von Personen entsprechen an deren Augen die Messdaten ermittelt wurden. Die Normaldaten können auch sämtliche Messdaten der Vergleichsgruppe enthalten, wobei dann die Verarbeitungseinrichtung eine genaue Abweichung der mit dem Sehprüfsystem ermittelten Messdaten vom fünfzigsten Perzentil der Vergleichsgruppe ausgeben kann. Beispielsweise ebenfalls eine Angabe welchem Perzentil der Vergleichsgruppe die Messdaten jeweils zugeordnet werden können. Unter der hier als Normalpopulation bezeichneten Vergleichsgruppe wird ein repräsentativer Bevölkerungsdurchschnitt verstanden.

Die Verarbeitungseinrichtung kann die gemessene zentrale Achslänge und/oder die periphere Achslänge, die Krümmung und die Refraktionseigenschaft des Auges jeweils mit den Normaldaten der zentralen Achslänge und/oder der peripheren Achslänge, der Krümmung und der Refraktionseigenschaft eines Auges vergleichen, wobei die Verarbeitungseinrichtung die Normaldaten für den Vergleich nach einer Übereinstimmung von zentraler Achslänge und/oder peripherer Achslänge, Krümmung der Refraktionseigenschaft mit den Messdaten der Messung auswählen kann.

Die Verarbeitungseinrichtung kann demnach nicht nur die jeweiligen Datensätze von Messdaten und Normaldaten für Achslängen, Krümmung und Refraktionseigenschaft unabhängig voneinander miteinander vergleichen, sondern auch aus der Datenbank beziehungsweise den Normaldaten ein Auge beziehungsweise die einem Auge zugehörigen Normaldaten beziehungsweise Messdaten auswählen die dem mit dem Sehprüfsystem gemessenen Messdaten am nächsten kommen beziehungsweise mit diesen übereinstimmen. Die Verarbeitungseinrichtung nimmt demnach einen Vergleich zwischen dem mit dem Sehprüfsystem gemessenen Auge und dem in der Datenbank in Form von Messdaten enthaltenen Augen vor und gibt diesen Vergleich aus. Eine untersuchende Person kann anhand des Vergleichs noch leichter beurteilen, inwieweit das mit dem Sehprüfsystem gemessene Auge von einer Norm abweicht oder bereits bekannte Symptome aufweist.

Die Verarbeitungseinrichtung kann bei dem Vergleich der Messdaten ein Alter des Probanden berücksichtigen. Gleichfalls kann die Datenbank mit den Normaldaten, Normaldaten enthalten, die jeweils einer Altersangabe zugeordnet werden können. Die Verarbeitungseinrichtung kann dann aus der Datenbank mit den Normaldaten die Normaldaten für den Vergleich auswählen, die dem Alter des Probanden entsprechen. Das Alter des

Probanden kann beispielsweise über Eingabemittel in die Verarbeitungseinrichtung vor oder nach einer Messung eingegeben werden. Aufgrund der bekannten Abhängigkeit einer Kurzsichtigkeit vom Alter wird durch die Berücksichtigung des Alters ein noch genauerer Vergleich der Messdaten möglich. Darüber hinaus ist es möglich, dass die Verarbeitungseinrichtung bei dem Vergleich der Messdaten beispielsweise eine Prävalenz der Kurzsichtigkeit in der Bevölkerung berücksichtigt. Neben dem Alter können dann auch Tätigkeitsprofile und Dispositionen berücksichtigt werden.

Als Normaldaten können Messdaten des Probanden mit einer peripheren und/oder zentralen Achslänge des Auges, einer Krümmung der Hornhaut des Auges und einer Refraktionseigenschaft des Auges, die zu einem vor der Messung liegenden Zeitpunkt bestimmt wurden, verwendet werden. So ist es möglich mit dem Sehprüfsystem zu unterschiedlichen Zeitpunkten bei ein und demselben Probanden eine Messung vorzunehmen und jeweils Messdaten zu gewinnen und zu speichern. Diese Messdaten können dann als Normaldaten für den Vergleich genutzt werden. So kann eine eventuelle Veränderung der optischen Eigenschaft des betreffenden Auges über einen Zeitabschnitt von beispielsweise Monaten oder Jahren bestimmt werden. Eine Ursache für einen sich beispielsweise verschlechternden Refraktionswert kann so noch einfacher ermittelt werden. Optional ist es aber auch möglich die zu jedem Messzeitpunkt gemessenen Messdaten stets mit Normaldaten einer Vergleichsgruppe zu vergleichen.

Die Verarbeitungseinrichtung kann die Messdaten der dritten Messvorrichtung mit den Messdaten der ersten Messeinrichtung und/oder der zweiten Messeinrichtung korrigieren. Die Bestimmung eines objektiven Refraktionswerts ist häufig schwierig und fehlerbehaftet, da wie vorstehend ausgeführt die Refraktionen von vielen Faktoren und Umgebungsbedingungen abhängig sein kann. Bei der Krümmung der Hornhaut und der Achslänge handelt es sich hingegen um Messwerte, die keinem Einfluss von beispielsweise Medikamenten oder einer Hirnaktivität unterliegen. Die Messdaten der dritten Messvorrichtung können daher mit den Messdaten der ersten und/oder zweiten Messeinrichtung vorteilhaft korrigiert werden.

So kann die Verarbeitungseinrichtung eine Plausibilitätsprüfung für einen gemessenen objektiven Refraktionswert des Auges mit der zentralen und/oder peripheren Achslänge und/oder mit der Krümmung der Hornhaut des Auges durchführen und bei einem abweichenden Refraktionswert den Refraktionswert nach der zentralen und/oder peripheren Achslänge und/oder der Krümmung der Hornhaut korrigieren. Die Plausibilitätsprüfung kann dabei anhand von Bereichsangaben erfolgen, die einen Toleranzbereich für die jeweiligen Messwerte angeben. Die Bereichsangaben können ebenfalls in der Datenbank gespeichert sein. Die Bereichsangaben können auf Erfahrungswerten basieren oder anhand einer Standartabweichung mit statistischen Mitteln bestimmt werden.

Das erfindungsgemäße Sehprüfsystem zum Überprüfen der Augen eines Probanden umfasst eine erste Messvorrichtung, eine zweite topografische Messvorrichtung, eine dritte refraktive Messvorrichtung und eine Verarbeitungseinrichtung, wobei mit der ersten Messvorrichtung eine zentrale Achslänge und eine periphere Achslänge eines Auges des Probanden messbar sind, wobei mit der zweiten Messvorrichtung eine Krümmung der Hornhaut des Auges messbar ist, wobei mit der dritten Messvorrichtung eine Refraktionseigenschaft des Auges messbar ist, wobei mit der Verarbeitungseinrichtung Messdaten der Messung der ersten, zweiten und dritten Messvorrichtung bearbeitbar sind, wobei das Sehprüfsystem eine Fixationseinrichtung aufweist, wobei das Auge mittels der Fixationseinrichtung wahlweise zur Messung der zentralen Achslänge oder der peripheren Achslänge relativ zum Sehprüfsystem fixierbar ist, wobei eine periphere Fixationsmarke der Fixationseinrichtung darstellbar und von dem Auge fixierbar ist, derart, dass eine Sehachse des Auges von einer Messachse der ersten Messvorrichtung abweicht. Zu den Vorteilen des erfindungsgemäßen Sehprüfsystems wird auf die Vorteilsbeschreibung des erfindungsgemäßen Verfahrens verwiesen.

Besonders vorteilhaft ist es, wenn die erste, zweite und dritte Messvorrichtung in einem Gerät integriert sind.

Die Fixationseinrichtung kann eine für das Auge im Unendlichen darstellbare und von dem Auge fokussierbare zentrale Fixationsmarke umfassen, wobei die zentrale Fixationsmarke derart angeordnet ist, dass bei einer Fokussierung der zentralen Fixationsmarke durch das Auge eine Sehachse des Auges mit einer optischen Messachse der ersten Messvorrichtung fluchten kann. Prinzipiell können die erste Messvorrichtung, die zweite Messvorrichtung und/oder dritte Messvorrichtung die Fixationseinrichtung aufweisen.

Die Fixationseinrichtung kann beispielsweise einen Bildschirm oder einen geeigneten Projektor umfassen, mit dem die zentrale Fixationsmarke für das Auge im Unendlichen sichtbar darstellbar ist. Die zentrale Fixationsmarke kann eine bildhafte Darstellung eines Gegenstands sein, sodass eine Fokussierung des Auges auf einen Punkt vermieden wird. Die zentrale Fixationsmarke kann in einem Strahlengang des Sehprüfsystems über beispielsweise einen Teilerwürfel eingekoppelt werden, so dass die zentrale Fixationsmarke für den Probanden sichtbar ist.

Die Fixationseinrichtung umfasst eine für das Auge darstellbare und von dem Auge fokussierbare periphere Fixationsmarke, wobei die periphere Fixationsmarke derart angeordnet sein kann, dass bei einer Fokussierung der peripheren Fixationsmarke durch das Auge eine Sehachse des Auges relativ zu einer optischen Messachse der ersten Messvorrichtung um einen Winkel α > 0° geneigt sein kann.

Die periphere Fixationsmarke kann durch zumindest eine Leuchtdiode ausgebildet sein, die relativ zu der optischen Messachse exzentrisch an einer dem Auge zugewandten Gehäuseseite des Sehprüfsystems angeordnet sein kann. Ein Proband kann dann vor der ihm zugewandten Gehäuseseite des Sehprüfsystems positioniert werden, wobei eine Augenbewegung eines Auges des Probanden leicht dadurch bewirkt werden kann, dass die Leuchtdiode in einigem Abstand von der optischen Messachse exzentrisch an der Gehäuseseite bzw. dem Gehäuse des Sehprüfsystems angeordnet ist und einen Lichtreiz erzeugt. Mit einer Leuchtdiode ist besonders kostengünstig eine Fixationsmarke ausbildbar. Weiter kann es auch vorgesehen sein, eine Mehrzahl von Leuchtdioden um die optische Messachse herum koaxial anzuordnen, um eine Bewegung des Auges in eine gewünschte Richtung zu initiieren. Darüber hinaus können auch mehrere Leuchtdioden in unterschiedlichen Abständen relativ zu der optischen Messachse an der Gehäuseseite angebracht sein, um ein Verschwenken des Auges relativ zu der optischen Messachse um unterschiedliche Winkel α zu bewirken. Gleichwohl kann die eine Leuchtdiode auch in ihrer Relativposition zur optischen Messachse verstellbar ausgebildet sein.

Die Fixationseinrichtung kann ein in einem Strahlengang der zentralen Fixationsmarke verschwenkbares optisches Umlenkelement umfassen, mit dem Strahlengang der zentralen Fixationsmarke derart umlenkbar ist, dass die zentrale Fixationsmarke als periphere Fixationsmarke relativ zu der optischen Messachse exzentrisch an einer dem Auge zugewandten Gehäuseseite des Sehprüfsystems darstellbar ist. Wenn beispielsweise die zentrale Fixationsmarke von einem Bildschirm oder einem anderen geeigneten Projektor ausgebildet ist, kann ein von dem Bildschirm ausgehender Strahlengang mit dem optischen Umlenkelement, welches beispielsweise ein Spiegel oder ein Prisma sein kann, so umgelenkt werden, dass der Strahlengang nicht mehr mit der optischen Messachse fluchtet bzw. parallel zu dieser verläuft. Das optische Umlenkelement kann beispielsweise ein verschiebbarer Kippspiegel sein, der in den Strahlengang der zentralen Fixationsmarke nach Bedarf ein- und ausgeschwenkt werden kann. Dann ist es im Übrigen auch möglich, dem Auge eine periphere Fixationsmarke darzubieten, die im Unendlichen fixierbar ist.

Die erste Messvorrichtung, die zweite Messvorrichtung und die dritte Messvorrichtung können eine gemeinsame Messachse aufweisen, die mit einer optischen Achse des Auges in Übereinstimmung bringbar sein kann. Dadurch das die Messvorrichtungen eine gemeinsame Messachse aufweisen wird eine besonders genaue und gut vergleichbare Gewinnung von Messdaten möglich. Ein Fluchten der gemeinsamen Messachse mit der optischen Achse des Auges ist insbesondere zur genauen Bestimmung einer Achslänge des Auges vorteilhaft.

Die zweite Messvorrichtung und/oder dritte Messvorrichtung kann eine Abstandsmesseinrichtung zur Messung eines Abstandes von Auge und zweiter Messvorrichtung und/oder dritter Messvorrichtung aufweisen. Die Abstandsmesseinrichtung kann den Abstand zwischen der refraktiven Messvorrichtung und/oder der topografischen Messvorrichtung und dem zu untersuchenden Auge messen. Dieser Abstand ist für eine Messdatenkorrektur wesentlich und es wird durch die Gewinnung von Abstandsdaten möglich den Probanden entsprechend korrekt vor dem Sehprüfsystem zu positionieren bzw. die mit der refraktiven Messvorrichtung gemessen Messdaten geeignet zur korrigieren. So kann vorteilhaft der Abstand zwischen der refraktiven Messvorrichtung und der Hornhaut des Auges, insbesondere der Vorderfläche der Hornhaut mit der Abstandsmesseinrichtung messbar sein. Darüber hinaus kann mit der Abstandsmesseinrichtung auch ein Abstand zu der Netzhaut bzw. Hinterfläche des Auges unter Einbeziehung der Achslänge bestimmt werden. Optional ist auch ein Abstand zu einer Hinterfläche der Hornhaut, Vorderfläche der Linse und/oder Hinterfläche der Linse bestimmbar. Die Bauart der Abstandsmesseinrichtung ist prinzipiell beliebig, wobei die Abstandsmesseinrichtung von der topografischen Messvorrichtung, einem Keratometer oder einem Scheimpflugsystem, ausgebildet werden kann.

Die erste Messvorrichtung kann eine Ultraschall-Messvorrichtung oder eine interferometrische Messvorrichtung sein. Prinzipiell kann die erste Messvorrichtung jede Art von Messvorrichtung sein, mit der eine zentrale Achslänge und eine periphere Achslänge eines Auges messbar sind.

Weiter kann die erste Messvorrichtung ein Interferometer zur optischen Kohärenzinterferometrie (OCT) sein.

Alternativ kann die erste Messvorrichtung ein Partial Coherence Interferometer (PCI) sein, wobei das Interferometer mit einer kohärenten Lichtquelle, zwei Messarmen und einer Detektoreinrichtung zur gleichzeitigen Erfassung der Vorderfläche der Hornhaut und der Netzhaut beziehungsweise einer optischen Grenzfläche des Auges eingerichtet sein kann. Dadurch, dass das Interferometer über zwei Messarme verfügt, ist es möglich die Vorderfläche des Auges und die Netzhaut gleichzeitig zu detektieren und einen Relativabstand von Vorderfläche und Netzhaut und damit die zentrale und/oder periphere Achslänge des Auges zu bestimmen. Dabei ist es unwesentlich, in welchem Abstand sich das Auge relativ zu dem Sehprüfsystem befindet, da die Abstandsmessung beziehungsweise Messung der Achslängen des Auges unabhängig zu dem Abstand vom Sehprüfsystem mit dem Interferometer durchgeführt werden kann. Eventuelle Messfehler des Interferometers aufgrund einer Abstandslage des Auges relativ zum Sehprüfsystem können so gänzlich ausgeschlossen werden. Die Achslängen werden damit besonders genau messbar. Neben den Achslängen können noch weitere optische Grenzflächen des Auges, wie Hinterfläche der Hornhaut, Vorderfläche der Linse, Hinterfläche der Linse und Abstände zwischen den optischen Grenzfläche mit dem Interferometer gemessen und von der Verarbeitungseinrichtung verarbeitet werden. Diese Messdaten können ebenfalls von der Verarbeitungseinrichtung für einen Vergleich mit entsprechenden Normaldaten herangezogen werden.

Die zweite Messvorrichtung kann einen Keratometer und/oder Scheimpflugsystem sein. Mit derartigen Messvorrichtungen ist es möglich eine Topografie bzw. die Krümmung der Hornhaut des Auges zu bestimmen.

Das Keratometer kann eine Beobachtungseinrichtung mit einer Kamera und mit der Kamera erfassbare Messmarken, die von einem kreisförmigen, nicht kollimierten Leuchtstreifen und zwei kollimierten Leuchtpunkten ausgebildet sein können, aufweisen. Eine Bauart der Messmarken des Keratometers ist grundsätzlich beliebig, wobei als Leuchtmittel Leuchtdioden vorgesehen werden können. Der Leuchtstreifen kann von einem kreisförmigen Lichtleiterelement erzeugt werden. Auch kann vorgesehen sein eine Mehrzahl konzentrischer, ringförmiger Leuchtstreifen zu erzeugen. Vorteilhaft kann als Licht für die Messmarken Infrarotlicht verwendet werden. Die Beobachtungseinrichtung kann eine Kamera sein, die über einen Teilerwürfel in einen Strahlengang des Sehprüfsystems bzw. des Keratometers eingekoppelt ist. Mit der Kamera kann ein Reflexbild der Messmarken auf der Hornhaut des Auges erfasst werden. Mittels Bildverarbeitung kann aus dem Reflexbild der Messmarken einfach eine Krümmung der Hornhaut abgeleitet und von der Verarbeitungseinrichtung dargestellt werden. Weiter ist es möglich die Kamera als Einrichtkamera bzw. Übersichtskamera für das Sehprüfsystem zur lagegenauen Anordnung und Ausrichtung des Auges des Probanden zu nutzen.

Das Scheimpflugsystem kann eine Projektionseinrichtung, die zur Beleuchtung des Auges mit einem Lichtspalt eingerichtet sein kann, und eine Beobachtungseinrichtung mit einer Kamera, welche zur Aufnahme eines Schnittbilds des Lichtspalts im Auge eingerichtet sein kann, aufweisen, wobei die Projektionseinrichtung und die Kamera nach der Scheimpflugregel relativ zueinander angeordnet sein können. Mit der Projektionseinrichtung kann dann der Lichtspalt auf das Auge projiziert werden, sodass eine Spaltbeleuchtung des Auges entlang der optischen Achse des Auges beziehungsweise der Sehachse erfolgen kann. Mit der nach dem Scheimpflugprinzip angeordneten Kamera kann das so erzeugte Schnittbild des Lichtspalts in dem Auge aufgenommen werden, sodass ein beleuchteter Querschnittsbereichs des Auges beziehungsweise ein vorderer Abschnitts des Auges optisch erfassbar sein kann. Ein sich so ergebenes Längsschnittbild des Auges kann dann vorzugsweise die optischen Grenzflächen der Hornhaut und der Linse wiedergeben. Aus einem so erhaltenen Bilddatensatz kann die Verarbeitungseinrichtung die Relativabstände der optischen Grenzflächen einfach berechnen. Weiter kann auch die Krümmung der Hornhaut einfach ermittelt werden. Das Scheimpflugsystem kann alleine oder auch zusammen mit dem Keratometer die zweite Messvorrichtung ausbilden.

Vorteilhaft kann die dritte Messvorrichtung ein Autorefraktometer sein.

Das Autorefraktometer kann eine Projektionseinrichtung, die zur Projektion eines Beleuchtungsmusters auf die Netzhaut des Auges eingerichtet sein kann, und eine Beobachtungseinrichtung, mit einer Beugungseinheit und einer Kamera, welche zur Aufnahme des Beleuchtungsmusters im Auge eingerichtet sein kann, aufweisen. Die Projektion des Beleuchtungsmusters kann dabei so erfolgen, dass das Beleuchtungsmuster auf der Netzhaut fokussiert ist. Mit der optischen Beobachtungseinrichtung kann das an der Netzhaut reflektierte Beleuchtungsmuster durch die Linse des Auges hindurch betrachtet werden, sodass ein Abbildungsmuster auf einem fotoelektrischen Sensor der Kamera abgebildet wird. Dieses Abbildungsmuster wird von der Kamera aufgenommen und mittels Bildbearbeitung ausgewertet. Entsprechend der Refraktionseigenschaften des Auges ist das auf die Netzhaut projizierte Beleuchtungsmuster charakteristisch verzerrt, sodass im Rahmen der Auswertung des Beleuchtungsmusters aus einem Maß der Verzerrung die Refraktionseigenschaften des Auges abgeleitet werden können. Die Beugungseinheit kann beispielsweise eine Lochblende mit einer Anzahl kreisförmig angeordneter Löcher sein, wobei über ein Ablenkprisma oder eine entsprechende Linse die Strahlengänge der Löcher jeweils auf den Sensor der Kamera abgelenkt werden können. Alternativ kann die Beugungseinheit auch ein diffraktives optisches Element (DOE) sein.

Weitere vorteilhafte Ausführungsformen eines Sehprüfsystems ergeben sich aus den Merkmalsbeschreibungen der auf den Verfahrensanspruch 1 rückbezogenen Unteransprüche.

Im Folgenden wird die Erfindung oder Bezugnahme auf die beigefügte Zeichnung näher erläutert.

Es zeigen:
- **Fig. 1**: eine schematische Darstellung eines Sehprüfsystems;
- **Fig. 2**: eine Schnittansicht eines Auges.

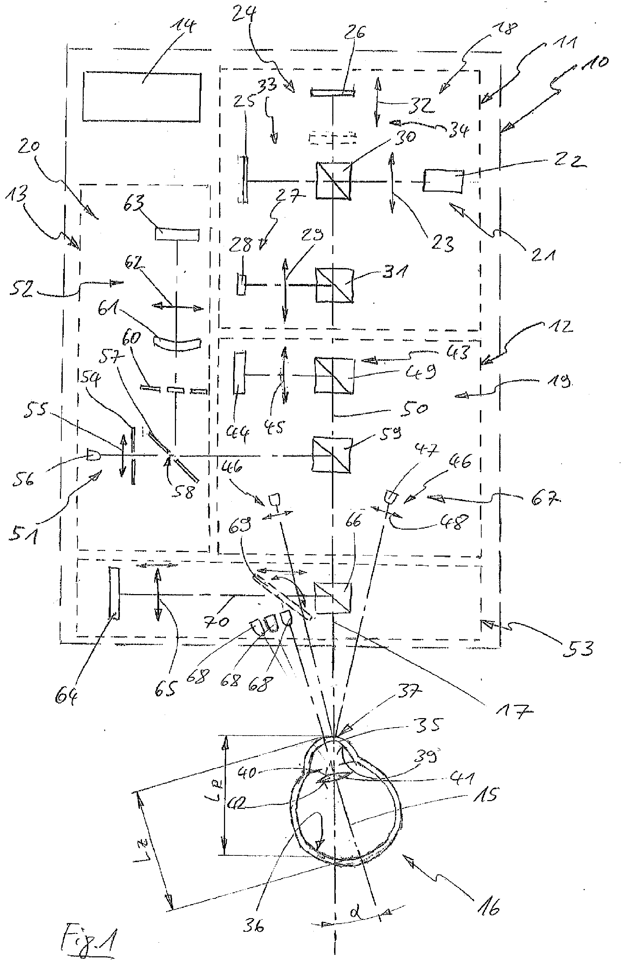

Die **Fig. 1** zeigt eine schematische Darstellung eines Aufbaus eines Sehprüfsystems 10 umfassend eine erste interferometrische Messvorrichtung 11, eine zweite topografische Messvorrichtung 12, eine dritte refraktive Messvorrichtung 13 und eine Verarbeitungseinrichtung 14.

Das Sehprüfsystem 10 ist relativ zu einer Sehachse 15 bzw. optische Achse eines zu untersuchenden Auges 16 so angeordnet, dass die Sehachse 15 mit einer Messachse 17 des Sehprüfsystems 10 nicht übereinstimmt bzw. abweicht. So ist die Sehachse 15 des Auges 16 relativ zu der optischen Messachse 17 um einen Winkel α geneigt. Die erste interferometrische Messvorrichtung 11 wird von einem Partial Coherence Interferometer 18, die zweite topografische Messvorrichtung 12 von einem Keratometer 19 und die dritte refraktive Messvorrichtung 13 von einem Autorefraktometer 20 ausgebildet.

Das Interferometer 18 ist im Wesentlichen aus einer Lasereinrichtung 21 mit einer Laserlichtquelle 22 und einer Linsenanordnung 23 , einer Spieleinrichtung 24 mit einem ersten Spiegel 25 und einem zweiten Spiegel 26, einer Detektoreinrichtung 27 mit einem Detektor 28 und einer Linsenanordnung 29 sowie einem ersten Teilerwürfel 30 und einem zweiten Teilerwürfel 31 ausgebildet. Insbesondere der zweite Spiegel 26 ist längs verschieblich entlang des Doppelpfeils 32 angeordnet, sodass eine Länge eines zweiten Referenzarms 34 beziehungsweise einer entsprechenden Referenzstrecke veränderlich ist. Ein erster Referenzarm 33 ist hingegen nicht längenveränderlich ausgebildet. Durch eine Verschiebung des zweiten Spiegels 26 können verschiedene, auf der Sehachse 15 liegende Bereiche des Auges 16 abgetastet werden. Insbesondere ist es möglich eine zentrale Achslänge (L_{Z}) und eine periphere Achslänge (L_{P}) des Auges 16 von der Hornhaut 35 bis zu der Netzhaut 36 beziehungsweise von einer Vorderfläche 37 der Hornhaut 35 bis zu einer Hinterfläche 38 der Netzhaut 36 zu messen. Auf eine weitergehende Erläuterung einer bekannten Funktion des Partial Coherence Interferometer 18 wird hier verzichtet. Weiter können auch Messdaten gewonnen werden, die Relativpositionen optischer Grenzflächen auf der Sehachse 15, wie die Vorderfläche 37 der Hornhaut 35, der Hinterfläche 39 der Hornhaut 35, eine Vorderfläche 40 einer Linse 41, einer Hinterfläche 42 der Linse 41 und die Hinterfläche 38 der Netzhaut 36, beschreiben.

Das Keratometer 19 umfasst eine Beobachtungseinrichtung 43 mit einer Kamera 44 und einer Linsenanordnung 45 sowie mit der Kamera 44 erfassbare Messmarken 46, die jeweils von einer Infrarotlichtquelle 47 und einer Linsenanordnung 48 ausgebildet sind. Die Infrarotlichtquelle kann beispielsweise eine Leuchtdiode sein. Die Messmarken 46 können zwei kollimierte Leuchtpunkte auf der Hornhaut 35 ausbilden, die mit der Kamera 44 erfassbar sind. Die Messmarken 46 sind hier mit einem kreisförmigen, nicht kollimierten Leuchtstreifen, der nicht dargestellt ist, ergänzt. Die Beobachtungseinrichtung 43 ist über einen Teilerwürfel 49 in einen Strahlengang 50 des Sehprüfsystems 10 eingekoppelt.

Das Autorefraktometer 20 dient zur Bestimmung der Refraktionseigenschaften des Auges 16 und umfasst im Wesentlichen eine Projektionseinrichtung 51 und eine Beobachtungseinrichtung 52 sowie eine Fixationseinrichtung 53, die aber auch unabhängig vom Autorefraktometer 20 ausgebildet sein kann. Mit der optischen Projektionseinrichtung 51 kann ein Beleuchtungsmuster auf die Netzhaut 36 des Auges 16 projiziert und dort fokussiert werden. Die Projektionseinrichtung 51 umfasst dabei eine Lochblende 54, eine Linsenanordnung 55 und eine Infrarotlichtquelle 56. Das Beleuchtungsmuster wird durch einen Spiegel 57 mit einer Lochblende 58 hindurch über einen ersten Teilerwürfel 59 des Autorefraktometers 20 in den Strahlengang 50 des Sehprüfsystems eingekoppelt. Die optische Beobachtungseinrichtung 52 umfasst eine 6-fach Lochblende 60, ein Ablenkprisma 61, eine Linsenanordnung 62 und eine Kamera 63. Die mit der Kamera 63 aufgenommenen Bilddaten werden in der Verarbeitungseinrichtung 14 verarbeitet und ausgewertet, um die Refraktionseigenschaften des Auges 16 zu bestimmen. Die Beobachtungseinrichtung 52 ist über den Spiegel 57 und den ersten Teilerwürfel 59 in den Strahlengang 50 eingekoppelt.

Die Fixationseinrichtung 53 des Autorefraktometers 20 ist aus einem Bildschirm 64 zur bildhaften Darstellung einer zentralen Fixationsmarke und einer Linsenanordnung 65 zur Darstellung der zentralen Fixationsmarke im Unendlichen ausgebildet. Die Linsenanordnung 65 ist in Richtung eines Strahlengangs 70 des Bildschirms 64 verschiebbar, um verschiedene Akkommodationszustände bzw. verschiedene Sehentfernungen des Auges 16 einstellen zu können. Ein zweiter Teilerwürfel 66 ermöglicht die Einkopplung der zentralen Fixationsmarke in den Strahlengang 50. Der erste Teilerwürfel 59 und der zweite Teilerwürfel 66 sind dann Bestandteil des Autorefraktometers 20.

Die Fixationseinrichtung 53 des Autorefraktometers 20 umfasst weiter eine für das Auge 16 darstellbare und vom dem Auge 16 fokussierbare periphere Fixationsmarke, die hier von einer der Leuchtdioden 68 ausgebildet werden kann. Die Leuchtdioden 68 sind relativ zu der optischen Messachse 17 exzentrisch an einer dem Auge 16 zugewandten, und hier nicht dargestellten Gehäuseseite des Sehprüfsystems 10 angeordnet. Zur Darstellung einer peripheren Fixationsmarke wird eine der Leuchtdioden 68 zum Leuchten gebracht, so dass aufgrund des erzeugten Lichtreizes eine Drehung des Auges 16 um den Winkel α mit der Sehachse 15 relativ der Messachse 17 bei gleichzeitiger Akkommodation auf die periphere Fixationsmarke erfolgt. So ist es dann möglich, mit dem Interferometer 18 die periphere Achslänge L_{P} des Auges zu messen. Je nach dem welche der Leuchtdioden 68 zur Darbietung der peripheren Fixationsmarke genutzt wird, kann die Messung der peripheren Achslänge L_{P} bei unterschiedlichen Neigungen der Sehachse 15 relativ zur Messachse 17 um den Winkel α erfolgen. Dass bei der Darstellung der peripheren Fixationsmarke eine Akkommodation des Auges in einem Nahbereich vorliegt, ist für die Messung der peripheren Achslänge A_{P} nicht von Bedeutung. Da vor oder nach der Messung der peripheren Achslänge A_{P} die zentrale Achslänge A_{Z} gemessen wird, kann die Verarbeitungseinrichtung 14 einen Vergleich der zentralen Achslänge A_{Z} mit der peripheren Achslänge A_{P} durchführen und ein Ergebnis des Vergleichs zusammen mit den übrigen Messdaten ausgeben. Je nach einem Verhältnis der Achslängen A_{P} zu A_{Z} kann eine untersuchende Person dann eine Refraktionseigenschaft des Auges 16 beurteilen.

Alternativ kann die Fixationseinrichtung 53 einen Kippspiegel 69 aufweisen, der in den Strahlengang 70 des Bildschirms 64 und der Linsenanordnung 65 bzw. der zentralen Fixationsmarke einschwenkbar ist. Der Strahlengang 70 kann dann mittels des Kippspiegels 69 noch vor dem zweiten Teilerwürfel 66 umgelenkt und auf das Auge 16 unter dem Winkel α relativ zu der Messachse 17 gerichtet werden. Die zentrale Fixationsmarke wird sodann dem Auge 16 als periphere Fixationsmarke dargeboten. Vorteilhaft ist hier, dass das Auge 16 die periphere Fixationsmarke auch im Unendlichen akkommodieren kann. Neben der Messung der peripheren Achslänge L_{P} ist dann auch die Messung einer Refraktionseigenschaft des Auges 16 mit dem Autorefraktometer 20 ohne Verabreichung von Zykloplegika möglich. Die Fixationseinrichtung 53 kann auch um die Messachse 17 drehbar ausgebildet sein, so dass die peripheren Fixationsmarke an nahezu beliebigen Stellen im Gesichtsfeld des Auges 16 darbietbar ist.

Weiter ist eine Abstandsmesseinrichtung 67 vorgesehen, die hier von dem Keratometer 19 ausgebildet ist. Die Abstandsmesseinrichtung 67 umfasst die Messmarken 46 und die Beobachtungseinrichtung 43.

Bei dem hier dargestellten Sehprüfsystem 10 erfolgt eine zeitgleiche Messung mit der ersten Messvorrichtung 11, der zweiten Messvorrichtung 12 und der dritten Messvorrichtung 13, wobei mit der Verarbeitungseinrichtung 14 Messdaten der Messung der ersten Messvorrichtung 11, der zweiten Messvorrichtung 12 und der dritten Messvorrichtung 13 verarbeitet werden, wobei die Verarbeitungseinrichtung 14 eine hier nicht dargestellte Datenbank mit Normaldaten aufweist, wobei mittels der Verarbeitungseinrichtung 14 ein Vergleich der Messdaten mit den Normaldaten durchgeführt und ein Ergebnis des Vergleichs ausgegeben wird.

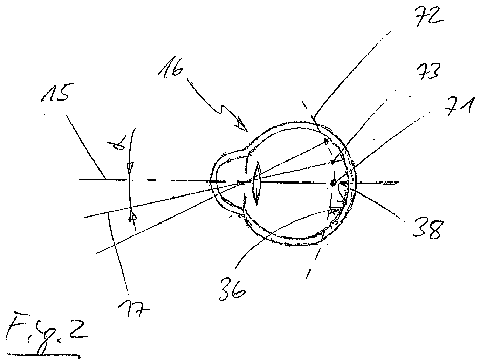

Die **Fig. 2** zeigt das Auge 16 mit der Sehachse 15 und einen auf der Sehachse 15 liegenden Fokus 71 eines scharfen Bildes in einer schalenförmigen Bildebene 72 bzw. Bildschale des Auges 16. Der Fokus 71 liegt hier vor der Hinterfläche 38 der Netzhaut 36, so dass das Auge 16 kurzsichtig ist. Bei einer um den Winkel α relativ zur Sehachse 15 verschwenkten Messachse 17 ergibt sich ein Fokus 73, der im Vergleich gegenüber dem Fokus 71 näher an der Hinterfläche 38 liegt. Hieraus ergibt sich eine Form der schalenförmigen Bildebene 72. Um eine optimale Korrektur der Kurzsichtigkeit des Auges 16 zu erzielen, ist es nun erforderlich nicht nur alleine den Fokus 71 mittels einer Sehhilfe oder eines invasiven Eingriffs auf die Hinterfläche 38 zu verschieben, sondern auch die schalenförmige Bildebene 72 entsprechend an die Form der Netzhaut 36 anzupassen. Diese Anpassung wird nur dann möglich, wenn Kenntnisse über die optischen Eigenschaften des Auges 16 im peripheren Bereich der Netzhaut 36 vorliegen. Um ein weiteres Fortschreiten einer Kurzsichtigkeit, beispielsweise bei Heranwachsenden, zu verhindern, kann die schalenförmige Bildebene 72 durch eine entsprechende Auswahl einer Sehhilfe über eine Krümmung der Netzhaut 36 hinausgehend enger gekrümmt werden. Neben den Refraktionseigenschaften des Auges 16 ist hier insbesondere eine Kenntnis von zentraler Achslänge L_{Z} und peripherer Achslänge L_{P} des Auges 16 von Bedeutung.

## Patentansprüche

1. Verfahren zum Überprüfen der Augen eines Probanden mit einem Sehprüfsystem (10), umfassend eine erste Messvorrichtung (11), eine zweite topografische Messvorrichtung (12), eine dritte refraktive Messvorrichtung (13) und eine Verarbeitungseinrichtung (14), wobei mit der ersten Messvorrichtung eine zentrale Achslänge (L_{Z}) und eine periphere Achslänge (L_{P}) eines Auges (16) des Probanden gemessen werden, wobei mit der zweiten Messvorrichtung eine Krümmung der Hornhaut (35) des Auges gemessen wird, wobei mit der dritten Messvorrichtung eine Refraktionseigenschaft des Auges gemessen wird, wobei mit der Verarbeitungseinrichtung Messdaten der Messung der ersten, zweiten und dritten Messvorrichtung verarbeitet werden, wobei die Verarbeitungseinrichtung die Messdaten ausgibt, wobei das Sehprüfsystem eine Fixationseinrichtung (53) aufweist,
**dadurch gekennzeichnet,**
**dass** das Auge mittels der Fixationseinrichtung wahlweise zur Messung der zentralen Achslänge (L_{Z}) oder der peripheren Achslänge (L_{P}) relativ zum Sehprüfsystem fokussiert wird, wobei eine periphere Fixationsmarke der Fixationseinrichtung dargestellt und von dem Auge fokussiert wird, derart, dass eine Sehachse (15) des Auges von einer Messachse (17) der ersten Messvorrichtung abweicht.

2. Verfahren nach Anspruch 1,
**dadurch gekennzeichnet,**
**dass** bei der Messung der zentralen Achslänge (L_{Z}) die Sehachse (15) des Auges (16) mit einer optischen Messachse (17) der ersten Messvorrichtung (11) fluchtet, wobei bei der Messung der peripheren Achslänge (L_{P}) die Sehachse des Auges relativ zu der optischen Messachse der ersten Messvorrichtung um einen Winkel α > 0° geneigt ist.

3. Verfahren nach Anspruch 2,
**dadurch gekennzeichnet,**
**dass** die Krümmung der Hornhaut (35) des Auges (16) und die Refraktionseigenschaft des Auges bei mit der optischen Messachse (17) der ersten Messvorrichtung (11) fluchtenden Sehachse (15) des Auges und/oder bei relativ zu der optischen Messachse der ersten Messvorrichtung um den Winkel α > 0° geneigter Sehachse des Auges gemessen wird.

4. Verfahren nach Anspruch 2 oder 3,
**dadurch gekennzeichnet,**
**dass** bei der Messung der peripheren Achslänge (L_{P}) die Sehachse (15) des Auges (16) relativ zu der optischen Messachse (17) der ersten Messvorrichtung (11) um einen Winkel α von 20°, ± 10° geneigt ist.

5. Verfahren nach einem der Ansprüche 2 bis 4,
**dadurch gekennzeichnet,**
**dass** die periphere Achslänge (L_{P}) für verschiedene Neigungen der Sehachse (15) des Auges (16) relativ zu der optischen Messachse (17) der ersten Messvorrichtung (11) gemessen wird, wobei der Winkel α in Schritten von 5° verändert wird.

6. Verfahren nach einem der vorangehenden Ansprüche,
**dadurch gekennzeichnet,**
**dass** mittels einer Fixationseinrichtung (53) des Sehprüfsystems (10) eine für das Auge (16) fokussierbare periphere Fixationsmarke dargestellt wird, wobei das Auge die Fixationsmarke fokussiert und eine Fixierung des Auges relativ zum Sehprüfsystem erfolgt.

7. Verfahren nach einem der vorangehenden Ansprüche,
**dadurch gekennzeichnet,**
**dass** mittels einer Fixationseinrichtung (53) des Sehprüfsystems (10) eine für das Auge (16) im Unendlichen fokussierbare zentrale Fixationsmarke dargestellt wird, wobei das Auge die Fixationsmarke fokussiert und eine Fixierung des Auges relativ zum Sehprüfsystem erfolgt.

8. Verfahren nach einem der vorangehenden Ansprüche,
**dadurch gekennzeichnet,**
**dass** mittels einer Fixationseinrichtung (53) des Sehprüfsystems (10) verschiedene Akkommodationszustände des Auges (16) des Probanden erzeugt werden.

9. Verfahren nach einem der vorangehenden Ansprüche,
**dadurch gekennzeichnet,**
**dass** an dem Auge (16) eine Messung mit der ersten, zweiten und dritten Messvorrichtung (11, 12, 13) zeitgleich durchgeführt wird.

10. Verfahren nach einem der vorangehenden Ansprüche,
**dadurch gekennzeichnet,**
**dass** mittels der Verarbeitungseinrichtung (14) ein Vergleich der zentralen Achslänge (L_{Z}) mit der peripheren Achslänge (L_{P}) durchgeführt und ein Ergebnis des Vergleichs zusammen mit den Messdaten ausgegeben wird.

11. Verfahren nach einem der vorangehenden Ansprüche,
**dadurch gekennzeichnet,**
**dass** mittels der Verarbeitungseinrichtung (14) ein Vergleich der zentralen Messdaten mit der peripheren Achslänge (L_{P}) durchgeführt und ein Ergebnis des Vergleichs zusammen mit den Messdaten ausgegeben wird.

12. Verfahren nach einem der vorangehenden Ansprüche,
**dadurch gekennzeichnet,**
**dass** mittels der Verarbeitungseinrichtung (14) durch den Vergleich eine Bestimmung eines Grades der Refraktion erfolgt.

13. Verfahren nach einem der vorangehenden Ansprüche,
**dadurch gekennzeichnet,**
**dass** mittels der Verarbeitungseinrichtung (14) aus den Messdaten eine Brechzahl und/oder ein Brechzahlgradient der Linse (41) des Auges (16) des Probanden bestimmt wird.

14. Verfahren nach einem der vorangehenden Ansprüche,
**dadurch gekennzeichnet,**
**dass** die Verarbeitungseinrichtung (14) eine Datenbank mit Normaldaten aufweist, wobei mittels der Verarbeitungseinrichtung ein Vergleich der Messdaten mit den Normaldaten durchgeführt und ein Ergebnis des Vergleichs ausgegeben wird.

15. Verfahren nach Anspruch 14,
**dadurch gekennzeichnet,**
**dass** als Normaldaten Messdaten von Augen einer Normalpopulation mit einer zentralen Achslänge (L_{Z}) und/oder einer peripheren Achslänge (L_{P}) eines Auges (16), einer Krümmung der Hornhaut (35) des Auges und einer Refraktionseigenschaft des Auges verwendet werden.

16. Verfahren nach Anspruch 14 oder 15,
**dadurch gekennzeichnet,**
**dass** die Verarbeitungseinrichtung (14) die gemessene zentralen Achslänge (L_{Z}) und/oder die periphere Achslänge (L_{P}), die Krümmung und die Refraktionseigenschaft des Auges (16) jeweils mit den Normaldaten der zentralen Achslänge (L_{Z}) und/oder der peripheren Achslänge (L_{P}), der Krümmung und der Refraktionseigenschaft eines Auges vergleicht, wobei die Verarbeitungseinrichtung die Normaldaten für den Vergleich nach einer Übereinstimmung von zentraler Achslänge (L_{Z}) und/oder peripheren Achslänge (L_{P}), Krümmung oder Refraktionseigenschaft mit den Messdaten der Messung auswählt.

17. Sehprüfsystem (10) zum Überprüfen der Augen eines Probanden, umfassend eine erste Messvorrichtung (11), eine zweite topografische Messvorrichtung (12), eine dritte refraktive Messvorrichtung (13) und eine Verarbeitungseinrichtung (14), wobei mit der ersten Messvorrichtung eine zentrale Achslänge (L_{Z}) und eine periphere Achslänge (L_{P}) eines Auges (16) des Probanden messbar sind, wobei mit der zweiten Messvorrichtung eine Krümmung der Hornhaut (35) des Auges messbar ist, wobei mit der dritten Messvorrichtung eine Refraktionseigenschaft des Auges messbar ist, wobei mit der Verarbeitungseinrichtung Messdaten der Messung der ersten, zweiten und dritten Messvorrichtung verarbeitbar sind, wobei das Sehprüfsystem eine Fixationseinrichtung (53) aufweist,
**dadurch gekennzeichnet,**
**dass** das Auge mittels der Fixationseinrichtung wahlweise zur Messung der zentralen Achslänge (L_{Z}) oder der peripheren Achslänge (L_{P}) relativ zum Sehprüfsystem fokussierbar ist, wobei eine periphere Fixationsmarke der Fixationseinrichtung darstellbar und von dem Auge fokussierbar ist, derart, dass eine Sehachse (15) des Auges von einer Messachse (17) der ersten Messvorrichtung abweicht.

18. Sehprüfsystem nach Anspruch 17,
**dadurch gekennzeichnet,**
**dass** die erste, zweite und dritte Messvorrichtung (11, 12, 13) in einem Gerät integriert sind.

19. Sehprüfsystem nach Anspruch 17 oder 18,
**dadurch gekennzeichnet,**
**dass** die Fixationseinrichtung (53) eine für das Auge (16) im Unendlichen darstellbare und von dem Auge fokussierbare zentrale Fixationsmarke umfasst, wobei die zentrale Fixationsmarke derart angeordnet ist, dass bei einer Fokussierung der zentralen Fixationsmarke durch das Auge eine Sehachse (15) des Auges mit einer optischen Messachse (17) der ersten Messvorrichtung (11) fluchtet.

20. Sehprüfsystem nach einem der Ansprüche 17 bis 19,
**dadurch gekennzeichnet,**
**dass** die periphere Fixationsmarke derart angeordnet ist, dass bei einer Fokussierung der peripheren Fixationsmarke durch das Auge die Sehachse (15) des Auges relativ zu der optischen Messachse (17) der ersten Messvorrichtung um einen Winkel α > 0° geneigt ist.

21. Sehprüfsystem nach Anspruch 20,
**dadurch gekennzeichnet,**
**dass** die periphere Fixationsmarke durch zumindest eine Leuchtdiode (68) ausgebildet ist, die relativ zu der optischen Messachse (17) exzentrisch an einer dem Auge (16) zugewandten Gehäuseseite des Sehprüfsystems (10) angeordnet ist.

22. Sehprüfsystem nach Anspruch 20,
**dadurch gekennzeichnet,**
**dass** die Fixationseinrichtung (53) ein in einen Strahlengang (70) der zentralen Fixationsmarke verschwenkbares optisches Umlenkelement (69) umfasst, mit dem der Strahlengang der zentralen Fixationsmarke derart umlenkbar ist, dass die zentrale Fixationsmarke als periphere Fixationsmarke relativ zu der optischen Messachse (17) exzentrisch an einer dem Auge (16) zugewandten Gehäuseseite des Sehprüfsystems (10) darstellbar ist.

23. Sehprüfsystem nach einem der Ansprüche 17 bis 22,
**dadurch gekennzeichnet,**
**dass** die erste Messvorrichtung (11), die zweite Messvorrichtung (12) und die dritte Messvorrichtung (13) eine gemeinsame Messachse (17) aufweisen, die mit der optischen Achse (15) des Auges (16) in Übereinstimmung bringbar ist.

24. Sehprüfsystem nach einem der Ansprüche 17 bis 23,
**dadurch gekennzeichnet,**
**dass** die erste Messvorrichtung (11) eine Ultraschall-Messvorrichtung oder eine interferometrische Messvorrichtung ist.

25. Sehprüfsystem nach Anspruch 24,
**dadurch gekennzeichnet,**
**dass** die erste Messvorrichtung (11) ein Interferometer zur optischen Kohärenzinterferometrie (OCT) ist.

26. Sehprüfsystem nach Anspruch 24,
**dadurch gekennzeichnet,**
**dass** die erste Messvorrichtung (11) ein Partial Coherence Interferometer (18) ist, wobei das Interferometer mit einer kohärenten Lichtquelle (22), zwei Messarmen (33, 34) und einer Detektoreinrichtung (27) zur gleichzeitigen Erfassung der Vorderfläche (37) und der Netzhaut (36) des Auges (16) eingerichtet ist.

27. Sehprüfsystem nach einem der Ansprüche 17 bis 26,
**dadurch gekennzeichnet,**
**dass** die zweite Messvorrichtung (12) ein Keratometer (19) und/oder ein Scheimpflugsystem ist.

28. Sehprüfsystem nach einem der Ansprüche 17 bis 27,
**dadurch gekennzeichnet,**
**dass** die dritte Messvorrichtung (13) ein Autorefraktometer (20) ist.

## Claims

1. A method for testing the eyes of a test person with the aid of a vision testing system (10), comprising a first measuring device (11), a second topographic measuring device (12), a third refractive measuring device (13) and a processing means (14), a central axial length (L_{Z}) and a peripheral axial length (L_{P}) of an eye (16) of the test person being measured with the aid of said first measuring device, a curvature of the cornea (35) of the eye being measured with the aid of said second measuring device, a refractive property of the eye being measured with the aid of said third measuring device, measurement data of the measurements of the first, second and third measuring device being processed with the aid of said processing means, said processing means outputting the measurement data, said vision testing system presenting a fixation means (53),
**characterised in that**
the eye is focused in relation to the vision testing system by means of the fixation means either for measuring the central axial length (L_{Z}) or the peripheral axial length (L_{P}), a peripheral fixation mark of the fixation means being displayed and focused on by the eye in such a manner that a visual axis (15) of the eye differs from a measurement axis (17) of the first measuring device.

2. The method according to claim 1,
**characterised in that**
during the measurement of the central axial length (Lz), the visual axis (15) of the eye (16) is aligned with an optical measurement axis (17) of the first measuring device (11), the visual axis of the eye being inclined by an angle α > 0° in relation to the optical measurement axis of the first measuring device during the measurement of the peripheral axial length (L_{P}).

3. The method according to claim 2,
**characterised in that**
the curvature of the cornea (35) of the eye (16) and the refractive property of the eye are measured while the visual axis (15) of the eye is aligned with the optical measurement axis (17) of the first measuring device (11) and/or while the visual axis of the eye is inclined by the angle α > 0° in relation to the optical measurement axis of the first measuring device.

4. The method according to claim 2 or 3,
**characterised in that**
the visual axis (15) of the eye (16) is inclined by an angle α of 20°, ± 10°, in relation to the optical measurement axis (17) of the first measuring device (11) during the measurement of the peripheral axial length (Lp).

5. The method according to any one of the claims 2 to 4,
**characterised in that**
the peripheral axial length (L_{P}) is measured for different inclines of the visual axis (15) of the eye (16) in relation to the optical measurement axis (17) of the first measuring device (11), the angle α being changed in 5° increments.

6. The method according to any one of the preceding claims,
**characterised in that**
a peripheral fixation mark that can be focused on by the eye (16) is displayed by means of a fixation means (53) of the vision testing system (10), the eye focusing on the fixation mark and the eye being fixated in relation to the vision testing system.

7. The method according to any one of the preceding claims,
**characterised in that**
a central fixation mark that can be focused on by the eye (16) at infinity is displayed by means of a fixation means (53) of the vision testing system (10), the eye focusing on the fixation mark and the eye being fixated in relation to the vision testing system.

8. The method according to any one of the preceding claims,
**characterised in that**
different accommodation states of the eye (16) of the test person are produced by means of a fixation means (53) of the vision testing system (10).

9. The method according to any one of the preceding claims,
**characterised in that**
a measurement of the eye (16) is carried out using the first, second and third measuring device (11, 12, 13) at the same time.

10. The method according to any one of the preceding claims,
**characterised in that**
by means of the processing means (14), a comparison of the central axial length (L_{Z}) to the peripheral axial length (L_{P}) is carried out and a result of said comparison is output together with the measurement data.

11. The method according to any one of the preceding claims,
**characterised in that**
by means of the processing means (14), a comparison of the central measurement data to the peripheral axial length (L_{P}) is carried out and a result of said comparison is output together with the measurement data.

12. The method according to any one of the preceding claims,
**characterised in that**
a degree of refraction is determined by means of the processing means (14) through the comparison.

13. The method according to any one of the preceding claims,
**characterised in that**
a refraction index and/or a refraction index gradient of the lens (41) of the eye (16) of the test person is/are determined from the measurement data by means of the processing means (14).

14. The method according to any one of the preceding claims,
**characterised in that**
the processing means (14) presents a database with normal data, a comparison of the measurement data to the normal data being carried out and a result of said comparison being output by means of said processing means.

15. The method according to claim 14,
**characterised in that**
measurement data of eyes of a normal population, with a central axial length (L_{Z}) and/or a peripheral axial length (L_{P}) of an eye (16), a curvature of the cornea (35) of the eye and a refractive property of the eye, are used as normal data.

16. The method according to claim 14 or 15,
**characterised in that**
the processing means (14) in each instance compares the measured central axial length (L_{Z}) and/or the peripheral axial length (L_{P}), the curvature and the refractive property of the eye (16) to the normal data of the central axial length (L_{Z}) and/or of the peripheral axial length (L_{P}), of the curvature and of the refractive property of an eye, said processing means selecting the normal data for the comparison according to a consistency of the central axial length (L_{Z}) and/or peripheral axial length (L_{P}), the curvature or the refractive property with the measurement data of the measurement.

17. A vision testing system (10) for testing the eyes of a test person, comprising a first measuring device (11), a second topographic measuring device (12), a third refractive measuring device (13) and a processing means (14), a central axial length (L_{Z}) and a peripheral axial length (L_{P}) of an eye (16) of the test person being measured with the aid of said first measuring device, a curvature of the cornea (35) of the eye being measured with the aid of said second measuring device, a refractive property of the eye being measured with the aid of said third measuring device, said processing means being configured to process measurement data of the measurements of the first, second and third measuring device, said vision testing system presenting a fixation means (53),
**characterised in that**
the eye is able to be focused in relation to the vision testing system by means of the fixation means either for measuring the central axial length (L_{Z}) or the peripheral axial length (L_{P}), a peripheral fixation mark of the fixation means being displayed and focused on by the eye in such a manner that a visual axis (15) of the eye differs from a measurement axis (17) of the first measuring device.

18. The vision testing system according to claim 17,
**characterised in that**
the first, second and third measuring device (11, 12, 13) are integrated in one piece of equipment.

19. The vision testing system according to claim 17 or 18,
**characterised in that**
the fixation means (53) comprises a central fixation mark that is configured to be displayed at infinity for the eye (16) and is configured to be focused on by the eye, said central fixation mark being disposed in such a manner that a visual axis (15) of the eye is aligned with an optical measurement axis (17) of the first measuring device (11) when the central fixation mark is being focused on by the eye.

20. The vision testing system according to any one of the claims 17 to 19,
**characterised in that**
the peripheral fixation mark is disposed in such a manner that the visual axis (15) of the eye is inclined by an angle α > 0° in relation to the optical measurement axis (17) of the first measuring device when the peripheral fixation mark is being focused on by the eye.

21. The vision testing system according to claim 20,
**characterised in that**
the peripheral fixation mark is realised by at least one light emitting diode (68) that is disposed, in relation to the optical measurement axis (17), eccentrically on a housing side of the vision testing system (10) facing the eye (16).

22. The vision testing system according to claim 20,
**characterised in that**
the fixation means (53) comprises an optical deflexion element (69) which is configured to be pivoted into an optical path (70) of the central fixation mark and using which the optical path of the central fixation mark can be deflected in such a manner that the central fixation mark can be displayed as a peripheral fixation mark, in relation to the optical measurement axis (17), eccentrically on a housing side of the vision testing system (10) facing the eye (16).

23. The vision testing system according to any one of the claims 17 to 22,
**characterised in that**
the first measuring device (11), the second measuring device (12) and the third measuring device (13) present a common measurement axis (17) that can be harmonised with the optical axis (15) of the eye (16).

24. The vision testing system according to any one of the claims 17 to 23,
**characterised in that**
the first measuring device (11) is an ultrasonic measuring device or an interferometric measuring device.

25. The vision testing system according to claim 24,
**characterised in that**
the first measuring device (11) is an interferometer for optical coherence interferometry (OCT).

26. The vision testing system according to claim 24,
**characterised in that**
the first measuring device (11) is a partial coherence interferometer (18), said interferometer being designed to have a coherent light source (22), two measuring arms (33, 34) and a detector means (27) for simultaneously capturing the front face (37) and the retina (36) of the eye (16).

27. The vision testing system according to any one of the claims 17 to 26,
**characterised in that**
the second measuring device (12) is a keratometer (19) and/or a Scheimpflug system.

28. The vision testing system according to any one of the claims 17 to 27,
**characterised in that**
the third measuring device (13) is an autorefractometer (20).

## Revendications

1. Procédé destiné au contrôle des yeux d'une personne examinée à l'aide d'un système d'examen de la vue (10), comprenant un premier dispositif de mesure (11), un deuxième dispositif de mesure topographique (12), un troisième dispositif de mesure réfractif (13) et une unité de traitement (14), une longueur axiale centrale (L_{Z}) et une longueur axiale périphérique (L_{P}) d'un oeil (16) de la personne examinée étant mesurées à l'aide du premier dispositif de mesure, une courbure de la cornée (35) de l'oeil étant mesurée à l'aide du deuxième dispositif de mesure, une propriété de réfraction de l'oeil étant mesurée à l'aide du troisième dispositif de mesure, des données mesurées provenant de la mesure du premier, deuxième et troisième dispositif de mesure étant traitées à l'aide de l'unité de traitement, l'unité de traitement émettant les données mesurées, le système d'examen de la vue présentant une unité de fixation (53),
**caractérisé en ce que**
l'oeil est focalisé par rapport au système d'examen de la vue moyennant l'unité de fixation pour mesurer la longueur axiale centrale (L_{Z}) ou la longueur axiale périphérique (L_{P}), une marque de fixation périphérique de l'unité de fixation étant affichée et l'oeil focalisant ladite marque de fixation périphérique, de telle manière qu'un axe visuel (15) de l'oeil diffère d'un axe de mesure (17) du premier dispositif de mesure.

2. Procédé selon la revendication 1,
**caractérisé en ce que**
lors de la mesure de la longueur axiale centrale (Lz), l'axe visuel (15) de l'oeil (16) est aligné avec un axe de mesure (17) optique du premier dispositif de mesure (11), l'axe visuel de l'oeil étant incliné d'un angle α > 0° par rapport à l'axe de mesure optique du premier dispositif de mesure lors de la mesure de la longueur axiale périphérique (L_{P}).

3. Procédé selon la revendication 2,
**caractérisé en ce que**
la courbure de la cornée (35) de l'oeil (16) et la propriété de réfraction de l'oeil sont mesurées lorsque l'axe visuel (15) de l'oeil est aligné avec l'axe de mesure (17) optique du premier dispositif de mesure (11) et/ou lorsque l'axe visuel de l'oeil est incliné de l'angle α > 0° par rapport à l'axe de mesure optique du premier dispositif de mesure.

4. Procédé selon la revendication 2 ou 3,
**caractérisé en ce que**
l'axe visuel (15) de l'oeil (16) est incliné d'un angle α qui se monte à 20°, ± 10°, par rapport à l'axe de mesure (17) optique du premier dispositif de mesure (11) lors de la mesure de la longueur axiale périphérique (L_{P}).

5. Procédé selon l'une quelconque des revendications 2 à 4,
**caractérisé en ce que**
la longueur axiale périphérique (L_{P}) est mesurée pour des inclinaisons différentes de l'axe visuel (15) de l'oeil (16) par rapport à l'axe de mesure (17) optique du premier dispositif de mesure (11), l'angle α étant adapté dans des étapes de 5°.

6. Procédé selon l'une quelconque des revendications précédentes,
**caractérisé en ce**
**qu'**une unité de fixation (53) du système d'examen de la vue (10) affiche une marque de fixation périphérique qui peut être focalisée par l'oeil (16), l'oeil focalisant la marque de fixation et étant fixé par rapport au système d'examen de la vue.

7. Procédé selon l'une quelconque des revendications précédentes,
**caractérisé en ce**
**qu'**une unité de fixation (53) du système d'examen de la vue (10) affiche une marque de fixation centrale qui peut être focalisée par l'oeil (16) à l'infini, l'oeil focalisant la marque de fixation et étant fixé par rapport au système d'examen de la vue.

8. Procédé selon l'une quelconque des revendications précédentes,
**caractérisé en ce**
**qu'**une unité de fixation (53) du système d'examen de la vue (10) génère des états d'accommodation différents de l'oeil (16) de la personne examinée.

9. Procédé selon l'une quelconque des revendications précédentes,
**caractérisé en ce que**
les mesures de l'oeil (16) sont relevées à l'aide du premier, deuxième et troisième dispositif de mesure (11, 12, 13) en même temps.

10. Procédé selon l'une quelconque des revendications précédentes,
**caractérisé en ce que**
l'unité de traitement (14) compare la longueur axiale centrale (L_{Z}) avec la longueur axiale périphérique (L_{P}) et émet un résultat de la comparaison conjointement avec les données mesurées.

11. Procédé selon l'une quelconque des revendications précédentes,
**caractérisé en ce que**
l'unité de traitement (14) compare les données mesurées centrales avec la longueur axiale périphérique (L_{P}) et émet un résultat de la comparaison conjointement avec les données mesurées.

12. Procédé selon l'une quelconque des revendications précédentes,
**caractérisé en ce que**
l'unité de traitement (14) détermine un degré de la réfraction au moyen de la comparaison.

13. Procédé selon l'une quelconque des revendications précédentes,
**caractérisé en ce que**
l'unité de traitement (14) détermine un indice de réfraction et/ou un gradient de l'indice de réfraction du cristallin (41) de l'oeil (16) de la personne examinée à partir des données mesurées.

14. Procédé selon l'une quelconque des revendications précédentes,
**caractérisé en ce que**
l'unité de traitement (14) présente une base de données comportant des données normales, l'unité de traitement comparant les données mesurées avec les données normales et émettant un résultat de la comparaison.

15. Procédé selon la revendication 14,
**caractérisé en ce que**
des données mesurées des yeux d'une population normale, comportant une longueur axiale centrale (L_{Z}) et/ou une longueur axiale périphérique (L_{P}) d'un oeil (16), une courbure de la cornée (35) de l'oeil et une propriété de réfraction de l'oeil, étant utilisées comme données normales.

16. Procédé selon la revendication 14 ou 15,
**caractérisé en ce que**
l'unité de traitement (14) compare la longueur axiale centrale (L_{Z}) mesurée et/ou la longueur axiale périphérique (L_{P}), la courbure et la propriété de réfraction de l'oeil (16) avec les données normales de la longueur axiale centrale (L_{Z}) et/ou la longueur axiale périphérique (L_{P}), de la courbure et de la propriété de réfraction d'un oeil, l'unité de traitement choisissant les données normales pour la comparaison selon une cohérence de la longueur axiale centrale (L_{Z}) et/ou la longueur axiale périphérique (L_{P}), la courbure ou la propriété de réfraction avec les données mesurées de la mesure.

17. Système d'examen de la vue (10) destiné au contrôle des yeux d'une personne examinée, ledit système d'examen de la vue comprenant un premier dispositif de mesure (11), un deuxième dispositif de mesure topographique (12), un troisième dispositif de mesure réfractif (13) et une unité de traitement (14), une longueur axiale centrale (L_{Z}) et une longueur axiale périphérique (L_{P}) d'un oeil (16) de la personne examinée pouvant être mesurées à l'aide du premier dispositif de mesure, une courbure de la cornée (35) de l'oeil pouvant être mesurée à l'aide du deuxième dispositif de mesure, une propriété de réfraction de l'oeil pouvant être mesurée à l'aide du troisième dispositif de mesure, des données mesurées provenant de la mesure du premier, deuxième et troisième dispositif de mesure pouvant être traitées à l'aide de l'unité de traitement, le système d'examen de la vue présentant une unité de fixation (53),
**caractérisé en ce que**
l'oeil est focalisé par rapport au système d'examen de la vue moyennant l'unité de fixation pour mesurer la longueur axiale centrale (L_{Z}) ou la longueur axiale périphérique (L_{P}), une marque de fixation périphérique de l'unité de fixation étant affichée et l'oeil focalisant ladite marque de fixation périphérique, de telle manière qu'un axe visuel (15) de l'oeil diffère d'un axe de mesure (17) du premier dispositif de mesure.

18. Système d'examen de la vue selon la revendication 17,
**caractérisé en ce que**
le premier, deuxième et troisième dispositif de mesure (11, 12, 13) sont intégrés dans un appareil.

19. Système d'examen de la vue selon la revendication 17 ou 18,
**caractérisé en ce que**
l'unité de fixation (53) comprend une marque de fixation centrale qui peut être affichée pour l'oeil (16) à l'infini et focalisée par l'oeil, la marque de fixation centrale étant disposée de telle manière qu'un axe visuel (15) de l'oeil est aligné avec un axe de mesure (17) optique du premier dispositif de mesure (11) lors d'une focalisation de la marque de fixation centrale par l'oeil.

20. Système d'examen de la vue selon l'une quelconque des revendications 17 à 19,
**caractérisé en ce que**
la marque de fixation périphérique est disposée de telle manière que l'axe visuel (15) de l'oeil est incliné d'un angle α > 0° par rapport à l'axe de mesure (17) optique du premier dispositif de mesure lors d'une focalisation de la marque de fixation périphérique par l'oeil.

21. Système d'examen de la vue selon la revendication 20,
**caractérisé en ce que**
la marque de fixation périphérique est réalisée par au moins une diode électroluminescente (68) qui est disposée excentriquement par rapport à l'axe de mesure (17) optique sur un côté de boîtier du système d'examen de la vue (10) qui est tourné vers l'oeil (16).

22. Système d'examen de la vue selon la revendication 20,
**caractérisé en ce que**
l'unité de fixation (53) comprend un élément déflecteur optique (69) qui peut être pivoté dans un trajectoire optique (70) de la marque de fixation centrale et avec lequel le trajectoire optique de la marque de fixation centrale peut être défléchi de telle manière que la marque de fixation centrale peut être affichée comme marque de fixation périphérique excentriquement par rapport à l'axe de mesure (17) optique sur un côté de boîtier du système d'examen de la vue (10) qui est tourné vers l'œil (16).

23. Système d'examen de la vue selon l'une quelconque des revendications 17 à 22,
**caractérisé en ce que**
le premier dispositif de mesure (11), le deuxième dispositif de mesure (12) et le troisième dispositif de mesure (13) présentent un axe de mesure (17) commun qui peut être harmonisé avec l'axe optique (15) de l'œil (16).

24. Système d'examen de la vue selon l'une quelconque des revendications 17 à 23,
**caractérisé en ce que**
le premier dispositif de mesure (11) est un dispositif de mesure par ultrasons ou un dispositif de mesure interférométrique.

25. Système d'examen de la vue selon la revendication 24,
**caractérisé en ce que**
le premier dispositif de mesure (11) est un interféromètre pour l'interférométrie à cohérence optique (OCT).

26. Système d'examen de la vue selon la revendication 24,
**caractérisé en ce que**
le premier dispositif de mesure (11) est un interféromètre à cohérence partielle (18), ledit interféromètre comportant une source lumineuse cohérente (22), deux bras de mesure (33, 34) et une unité détecteur (27) pour examiner en même temps la face antérieure (37) et la rétine (36) de l'oeil (16).

27. Système d'examen de la vue selon l'une quelconque des revendications 17 à 26,
**caractérisé en ce que**
le deuxième dispositif de mesure (12) est un kératomètre (19) et/ou un système de Scheimpflug.

28. Système d'examen de la vue selon l'une quelconque des revendications 17 à 27,
**caractérisé en ce que**
le troisième dispositif de mesure (13) est un autoréfractomètre (20).
